# EUROPEAN PATENT SPECIFICATION

(11) **EP 1 776 162 B1**
(45) Date of publication and mention of the grant of the patent: **30.11.2011**
(21) Application number: 05777610.6
(22) Date of filing: 28.07.2005
(51) Int. Cl.: A61K 8/04, A61Q 9/02

(54) **SELF-HEATING SHAVE FOAM PRODUCT**
SELBSTERWÄRMENDES RASIERSCHAUMPRODUKT
PRODUIT DE MOUSSE A RASER AUTO-CHAUFFANT

(30) Priority: 09.08.2004 US 914427
(43) Date of publication of application: 25.04.2007
(73) Proprietor: The Gillette Company, Boston, MA 02199-4099 (US)
(72) Inventor: XU, Yun, Andover, MA 01810 (US); OBIAS, Honorio, V., Medford, MA 02155 (US); NOVIKOV, Alexander, Framingham, MA 01701 (US); BARNET, Alfred, G., Hingham, MA 02043 (US); THONG, Stephen, H., Pennington, NJ 08534 (US)
(74) Representative: Chandrani, Vandita
(86) International application number: PCT/US2005/026827
(87) International publication number: WO 2006/020418

(56) References cited:
- WO-A-2004/075869
- US-A- 3 341 418
- US-A- 3 819 524
- US-A- 4 439 416

## Description

This invention relates to self-heating shave foam products.

Currently, a widely used and well-known form of shaving preparation is the type referred to as a shave foam. Such compositions generally take the form of an oil-in-water emulsion in which a foaming agent, generally a volatile (i.e., low boiling point) aliphatic hydrocarbon, is solubilized in the oil phase, and the water phase comprises a water-dispersible soap or interrupted soap component. The product is dispensed as a foam lather generated by the volatilization of the volatile hydrocarbon foaming agent. In general, the product requires shaking so that the oil phase and water phase are well mixed before dispensing.

Users of wet-shave razors generally appreciate a feeling of warmth against their skin during shaving. The warmth feels good, and also causes the user's skin to hydrate and beard to soften, resulting in a more comfortable shave. Various attempts have been made to provide a warm feeling during shaving. For example, soap-based shaving foams have been formulated to react exothermically upon release from the shaving canister, so that the foam imparts warmth to the skin, for example, as described in U.S. 3,341,418; U.S. 3,772,203; U.S. 3,819,524; U.S 3,866,800; and U.S. 3,878,118.

US-A-4 439 416 discloses a self-heating, self-foaming shaving cream compositions comprising a first and a second compartment. The second compartment comprises 12% of glycerylaldehyde, 18% of water and 65,5% of a shaving cream bases which itself comprises 88% of water. The total amount of water in the second compartment arises to 75%. The amount of volatile foaming agent is 4,5% for the first and the second compartment. The first compartment comprises a hydride compound dispersed in 85% of heavy paraffin oil with out any water present.

The intermediate international publication WO 2004/075869 discloses a post-foaming shave gel product comprising a first and a second compartment. The volatile foaming hydrocarbon such as n- or isobutane have a pressure of 3-20 psig at 20°C. The first compartment comprises the oxidant whereas the second compartment comprises the reductant. Both compartments comprise an oil-in-water emulsion having 55-90% water, 3-20% nonionic surfactant and 1-6% of a volatile self-foaming agent. The volatile agent is the same propellant as in the present application however differs in the in the pressure range.

The invention features a self-heating shave foam product including a container with two separate chambers, one of which contains an oxidant component, and the other of which contains a reductant component. The container also has at least one dispensing valve for dispensing the contents of the chambers. The oxidant component includes a first shave foam base and an oxidizing agent, and the reductant component includes a second shave foam base and a reducing agent. The shave foam bases each independently include an oil in-water emulsion including water, a volatile foaming agent with a vapor pressure of 276 kPa (40 psig) to 483 kPa (70 psig) at 21°C, and a water-dispersible surface active agent comprising a non-ionic surfactant. The amount and proportion of the oxidizing agent and the reducing agent are selected to provide an exothermic reaction with a desirable heat profile upon mixing of the oxidant component and the reductant component during use of the shave foam product. The amount of water in each component comprises independently from each other from 55% to 95%, 1%-6% of volatile foaming agent and 2%-15% of non-ionic surfactant.

Preferably, at least one, or more preferably both, of the shave foam bases, are substantially free of soap and ionic surfactant (e.g., anionic surfactant). By "substantially free" is meant that the shave foam base contains less than 2% of soap and ionic surfactant, preferably less than 1.5% of soap and ionic surfactant, more preferably less than 1% of soap and ionic surfactant, and most preferably 0% of soap and ionic surfactant.

The non-ionic, substantially soap-free formulation is compatible with, and stable in the presence of, the active agents that are used to generate the warm sensation. The non-ionic shave foam base may also offer additional advantages such as alleviating the problems associated with soap-based products. The non-ionic surfactant can include a blend of two surfactants, one of the surfactants being more hydrophobic than the other. Typically, the surfactant blend may include fatty alcohol ethoxylates having relatively longer and shorter polyethylene oxide chains (polyoxyethylene chains). For example, the blend may include a fatty alcohol ethoxylate having from 2 to 20 ethoxy groups, and a fatty alcohol ethoxylate having from 21 to 100 ethoxy groups, provided in a ratio in the range of from 2.5:1 to 1:2.5.

Preferably, the first shave foam base and the second shave foam base are substantially identical, by which is meant that each shave foam base has at least three, preferably at least four, more preferably at least five, ingredients identical to those in the other shave foam base and, most preferably, such ingredients are present in approximately the same proportions as in the other shave foam base.

In another aspect, the invention features a method of using the above-described shave foam product. The method includes dispensing the shave foam from a container without shaking the container prior to or during dispensing, and contacting the oxidant component with the reductant component to provide an exothermic reaction. The method can further include applying the shave foam to the skin.

Some implementations can exhibit one or more of the following advantages. The shave foam product has a relatively long shelf stability, preferably from about one year to about three years. In some embodiments, the shave foam product does not require shaking to produce a shave foam. In some implementations, the foaming agent and other ingredients of the shave foam product remain emulsified and stabilized during storage in the container, rather than phase-separating into a heterogeneous mixture. This can eliminate the need for shaking the shave foam products prior to use, which shaking could be disadvantageous in a two-component container. The shave foam product provides a pleasant, warm feeling to the user before and during shaving, in combination with the aesthetic properties of a foam. The heating effect of the shave foam helps to hydrate a user's hair (e.g., beard) and prepare the hair for shaving, improving user comfort. The shave foam product is shelf stable (e.g., their contents do not phase-separate in their containers), and dispenses from the container in an attractive, aesthetic form. After dispensing, the shave foam provides a smooth, creamy, stable lather that develops quickly when the foam is spread over the skin. After being applied to a user's skin (e.g., to the user's face), the lather remains on the skin during shaving (e.g., the lather does not run off of the face), even when the foam is heated by the exothermic reaction. The lather helps to soften hair (e.g., beard hair) and protects the skin during shaving. The lather remains creamy and stable when the shave foam composition is heated. The shave foam provides desirable performance properties such as lubricity and skin-friendliness, which are maintained during and after heating. The chemistry of the heating system that is used to heat the shave foam is safe for use on the skin and does not irritate the skin. After shaving, the lather can be relatively easily removed from the skin.

Other features and advantages of the invention will be apparent from the description and from the claims.

As used in this application (unless indicated otherwise), all percentages are by weight on a solids basis.

The shave foam composition is divided into two separate components, (a) an oxidant component containing a first shave foam base and the oxidizing agent, and (b) a reductant component containing a second shave foam base and the reducing agent. Any ingredients that could be easily oxidized by the oxidizing agent during the product shelf life are included in the reductant component. These two components are maintained separate in the packaging of the shave foam composition, as will be discussed further below, and are mixed during or after dispensing. When the two components are mixed, an exothermic reaction occurs that heats the shave foam composition. If the exothermic reaction generates an acid that might tend to irritate the user's skin, then one component (preferably the reductant component) generally includes a neutralizing agent to neutralize this acid.

The shave foam base for use in the present invention includes water, a water-dispersible surface active agent which comprises a non-ionic surfactant, and a volatile foaming agent with a vapor pressure of 40 psig or more at 21°C. Preferably, the water-dispersible surface active agent consists essentially of a non-ionic surfactant. Preferably, the non-ionic surfactant includes a blend of non-ionic surfactants, more preferably a blend of a relatively hydrophobic non-ionic surfactant and a relatively hydrophilic non-ionic surfactant. A more preferred shave foam base includes, in addition to the aforementioned ingredients, one or more (or, in a most preferred embodiment, all) of the following optional ingredients: a water-soluble polymer, a fatty alcohol, an amphoteric surfactant, an emollient (e.g., an oil), and a thickener. The shave foam base is typically in the form of an oil-in-water emulsion.

The water-dispersible surface active agent, which is preferably a blend of surfactants, is selected to provide several functions. It functions as an emulsifier, solubilizer, detergent, and spreading or dispersing agent. First, the surfactants provide an emulsion that is stable during the shelf life of the product, allowing the product to be dispensed as a shave foam composition exhibiting little or no phase separation. Second, the surfactants provide lathering during foaming. Third, the surfactants are capable of providing a lather that will remain stable at elevated temperatures, i.e., the temperatures the shave foam composition can reach during heating, typically from 35°C to 50°C. By "stable," it is meant that the shave foam will not puddle in the user's hand or drip from the user's face, but will instead maintain substantially the same consistency before, during and after heating. The blend of surfactants is preferably present in both the oxidant and reductant components, so that both components can be provided as stable emulsions that can be dispensed in foam form.

The water-dispersible surface active agent includes a non-ionic surfactant, more preferably a blend of two or more non-ionic surfactants. Because they are stable in the presence of mild acids and alkalis, non-ionic surfactants can provide flexibility of formulation that is generally not possible using soaps. Preferred non-ionic surfactants include polyethoxylated fatty alcohol ethers. These are derived from fatty alcohols with C12-C24, preferably C12-C20, hydrocarbon chains (with a degree of unsaturation of 0-2) ethoxylated with 2 to 150, preferably 2 to 100, ethylene oxide units (i.e., ethoxy groups). Thus, one or more of the fatty alcohol ethoxylates can have the general formula: 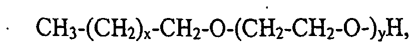 where x = 10-22 (preferably 10-18), y = 2-150 (preferably 2-100).. Examples of such surfactants include Steareth-21, Steareth-100, Myreth-4, Myreth-10, Laureth-4, and Laureth-35. In some embodiments, the water-dispersible surface active agent can include a mixture of non-ionic surfactants having long (C16-C24) hydrocarbon chains and/or non-ionic surfactants having medium (C12-C14) hydrocarbon chains.

The blend of fatty alcohol ethoxylates can include:
(a) at least one fatty alcohol ethoxylate with a long polyethylene oxide chain length (i.e., 21-150, preferably 21-100, ethylene oxide units or ethoxy groups) and at least one fatty alcohol ethoxylate with a short polyethylene oxide chain length (i.e., 2-20 ethylene oxide units or ethoxy groups); and/or
(b) at least one fatty alcohol ethoxylate with a long polyethylene oxide chain length and a different fatty alcohol ethoxylate with a long polyethylene oxide chain length; and/or
(c) at least one fatty alcohol ethoxylate with a short polyethylene oxide chain length and a different fatty alcohol ethoxylate with a short polyethylene oxide chain length.

Preferred long polyethylene oxide chain length fatty alcohol ethoxylates include Steareth-100 (100 indicates the polyethylene oxide chain length) and Steareth-21. Other long polyethylene oxide chain length fatty alcohol ethoxylates can be used, such as Ceteth-100, Oleth-100, Myreth-100, and Beheneth-100. These long polyethylene oxide chain length fatty alcohol ethoxylate surfactants have a preferred HLB range from 15 to 18. Suitable shorter polyethylene oxide chain length fatty alcohol ethoxylates include, for example, Steareth-2, Steareth-10, Ceteth-10, Ceteth-20, Steareth-20, Myreth-20, Oleth-20 and Beheneth-20. These shorter polyethylene oxide chain length fatty alcohol ethoxylate surfactants have a preferred HLB range from 4 to 16.

The short and long polyethylene oxide chain length surfactants are included in a ratio that provides the desired aesthetic and performance properties to the foam. Surfactant levels and types may be selected based on HLB matching of the ingredients (minus surfactants) with the HLB of the surfactant system. It is preferred to use a blend of high and low HLB surfactants to accomplish this. For example Steareth-2 (HLB 4.9) and Stereath-100 (HLB 18.8) can be blended to give an HLB of about 15. The surfactant level level may be further optimized to provide desired stability and formulation aesthetics. Thus, the relative amounts of the two surfactants may be adjusted to obtain a desired balance of properties. For a shave foam with good consistency and lathering, a suitable ratio of the short polyethylene oxide chain (more hydrophobic) surfactant to the long polyethylene oxide chain (more hydrophilic) surfactant would generally be in the range of about 2.5:1 to about 1:2.5, respectively.

In addition to or as an alternative to ethoxylated surfactants, suitable surfactant blends can include one or more non-ethoxylated surfactants, such as fatty ether or ester surfactants (e.g., polyglyceryl fatty esters, sugar ethers, sugar esters, esters of sugar derivatives). Examples of polyglyceryl fatty esters include decaglyceryl dipalmitate, decaglyceryl oleate, decagylceryl stearate, hexaglyceryl monostearate/oleate, decaglyceryl myristate, hexaglyceryl myristate, decagylceryl laurate, hexaglyceryl laurate, and triglyceryl stearate. Examples of sugar ethers include cetearyl polyglucoside, behenyl polyglucoside, myristyl polyglucoside, and cocoyl polyglucoside. Examples of sugar esters include sucrose esters, such as sucrose monostearate and sucrose distearate. Examples of esters of sugar derivatives include sorbitan esters, such as sorbitan monostearate, sorbitan palmitate, sorbitan oleate, sorbitan sesquioleate, and sorbitan isostearate. Generally, such non-ethoxylated surfactants will be included in the reductant component unless they are also determined to be stable in the presence of oxidizing agent.

The total amount of the water-dispersible surface active agent in either or each shave foam base (or in the composition as a whole) is generally in the range of from 2% to 15%, preferably from 3% to 12%. Including too high a level of the surfactants may result in inefficient mixing of the shave foam bases, which can limit the exothermic reaction between the shave foam bases, thereby reducing the warmth of the shave foam. Including too low a level of the surfactants may result in instability of the shave foam composition. The blends of short and long chain length fatty alcohol ethoxylates discussed above can stabilize the oil droplets, which are distributed in the water phase and in which the blowing gas (foaming agent) resides.

In certain embodiments, the oxidant component of a shave foam composition may include from 2% to 8%, preferably from 2% to 6% of a non-ionic surfactant. In certain embodiments, the oxidant component may include from 1% to 6%, preferably from 2% to 4%, of a shorter polyethylene oxide chain length non-ionic surfactant, such as Steareth-2. Alternatively or additionally, the oxidant component may include from 1% to 6%, preferably from 2% to 4% of a long polyethylene oxide chain length non-ionic surfactant, such as Steareth-21. In some embodiments, the oxidant component may include from 1% to 6% of one non-ionic surfactant, and from 1% to 6% of another, different, non-ionic surfactant.

In certain embodiments, the reductant component of a shave foam composition may include from 2% to 12%, preferably from 3% to 8% of a non-ionic surfactant, such as Steareth-100 or Steareth-21. The reductant component may include more than one type of non-ionic surfactant. For example, the reductant component may include from 1% to 8% of one non-ionic surfactant (e.g., Steareth-21), and from 1% to 8% of another, different, non-ionic surfactant (e.g., Steareth-100).

Water is the major component of the composition and is used in sufficient quantities to solubilize or disperse the surfactant components and form the continuous phase of the oil-in-water emulsion, while providing a stable foam of suitable viscosity with desirable lathering and rinsing properties. It is added in a sufficient quantity (q.s) to bring the total of all components to 100%. The composition (i.e., each of the oxidant and reductant components independently) typically includes from 55% to 95%, preferably from 60% to 90%, more preferably 65% to 85%, water. In certain embodiments, the oxidant component of the shave foam composition can include 60% to 90%, preferably 70% to 85%, water. In some embodiments, the reductant component can include from 55% to 90%, preferably from 70% to 85%, water. In certain embodiments (e.g., embodiments in which the reductant component includes a triethanolamine neutralizer), the reductant component may include from 58% to 70% water.

Each shave foam base includes a volatile foaming agent to expel the product and create a foam lather upon dispensing. The foaming agent can be any volatile hydrocarbon or halohydrocarbon with a sufficiently low boiling point. The typical boiling point of such an agent generally falls within the range of from about 30°C to about 40°C, preferably from about -25°C to about 10°C. Preferred foaming agents are selected from saturated aliphatic hydrocarbons or halohydrocarbons having from 3 to 6 carbon atoms such as n-pentane; isopentane; neopentane; n-butane; isobutanes; 1,1-difluoroethane; propane; and mixtures thereof. Most preferred are 1,1-difluoroethane (e.g., Dymel-152a) and a mixture of isobutane and propane in a weight ratio (isobutane:prapane) of from about 1:0 to about 7:1, preferably from about 3:1 to about 6:1 (e.g., Diversified A-46). The foaming agent will typically be selected so as to have a vapor pressure of about 276 kPa to 483 kPa (40 to 70 psig) at 21°C, preferably from about 310 kPa to 448 kPa (45 to 65 psig) at 21°C. The foaming agent is generally included in both the oxidant and reductant components in an amount of from 1% to 6%, preferably from 2% to 5%, more preferably from 3% to 4.5%, and may be added to concentrates formed by pre-mixing the other ingredients of each component.

It is preferred that one or both share foam bases include an emollient, to provide desirable cosmetic properties. The oil phase of the emulsion can include any desired emollient that is safe for use in a shave foam composition, is compatible with the other ingredients of the composition, and provides the desired aesthetics and in-shave lubricity. Suitable emollients include mineral oil, petrolatum, squalane/squalene, hydrogenated/unsaturated polyisobutene and mixtures thereof. These emollients are suitable for use with the surfactant blends discussed above. Preferably, the composition contains from 0.25% to 15% of the emollient, more preferably from 0.5% to 12% of the emollient, and most preferably from 0.75% to 8% of the emollient. The emollient is preferably included in both shave foam bases. In some embodiments, the oxidant component of a shave foam composition can include from 1% to 4% of an emollient (e.g., mineral oil). In certain embodiments, the reductant component of a shave foam composition can include from 1% to 5% of an emollient (e.g., mineral oil).

A thickener is optionally included to improve the consistency and stability of the shave foam, as well as to adjust its viscosity. The thickener also generally provides body to the shave foam. The thickener may be a water-soluble thickener, a water-insoluble thickener, or a mixture thereof. In some embodiments, either or both (preferably both) components of a shave foam composition can include from 0.01% to (15%, preferably 0.1% to 11%, of a thickener. In certain embodiments, the oxidant component and/or the reductant component of a shave foam composition can each include from 1% to 10%, preferably from 3% to 6% of a thickener. The thickener included in the oxidant component must, of course, be stable in the presence of an oxidizing agent (such a thickener includes, for example, polyvinylpyrrolidone).

A preferred thickener is a fatty alcohol (which is a water-insoluble thickener). Suitable fatty alcohols have a chain length of 12-22 carbon atoms, and a degree of unsaturation of 0-1. Suitable fatty alcohols include, for example, myristyl alcohol, lauryl alcohol, cocoyl alcohol, cetyl alcohol, cetearyl alcohol, oleyl alcohol, stearyl alcohol and behenyl alcohol. Generally the composition can include from 0% to 15%, preferably 0.1% to 15%, more preferably 1% to 15%, most preferably 2% to 8%, of a fatty alcohol thickener.

Other examples of suitable water-insoluble thickeners include ethoxylated or non-ethoxylated fatty esters (e.g., PEG-150 distearate, PEG-150 pentaerythrityl tetrastearate, pentaerythrityl tetraisostearate, pentaerythrityl tetrastearate, isostearyl neopentanoate, and mixtures thereof). In some embodiments, the oxidant component and/or the reductant component of a shave foam composition can include from 0.1% to 1% of such a thickener, such as PEG-150 distearate.

In addition to, or in some cases instead of, the fatty alcohol thickener, the composition may include other thickeners. Examples of other suitable thickeners include water-soluble thickeners, such as hydroxyalkyl cellulose polymers, e.g., hydroxyethyl cellulose and hydroxypropyl cellulose (sold under the trademarks "Natrosol" and "Klucel" respectively), carboxymethyl cellulose, cellulose methyl ether (sold under the trademark "Methocel"), hydroxypropyl starch phosphate (sold under the trademark "Structure XL"), other polysaccharides such as xanthan gum, guar gum, modified starch and carageenan, and mixtures thereof. In some embodiments, the reductant component of a shave foam composition may advantageously include up to 1% of a water-soluble thickener, such as hydroxyethyl cellulose.

As a thickener and/or for increased lubricity, the shave foam composition can also include a lubricious water-soluble polymer. Such polymers typically have a molecular weight of between about 300,000 daltons and about 15,000,000 daltons. Suitable polymers include, for example, polyvinylpyrrolidone (PVP), PVP/vinyl acetate copolymer, polyethylene oxide, polyacrylamide, and mixtures thereof. If a lubricious water-soluble polymer is included, it is typically provided in the shave foam composition in an amount of from 0.005% to 4%, preferably from 0.01% to 1.5%, of the composition. In some embodiments, a reductant component of a shave foam composition may advantageously include up to 1% of a lubricious water-soluble polymer, such as polyacrylamide.

As discussed above, the heating reagents generally include an oxidizing agent, included in the oxidant component, and a reducing agent, included in the reductant component. Suitable oxidizing agents include peroxides, such as hydrogen peroxide (typically added as a 35% solution), benzoylperoxide, peroxomonosulfate, peroxodisulfate, urea hydrogen peroxide, and t-butyl peroxide. In some embodiments, the oxidant component of a shave foam composition may include from 2% to 10% of an oxidizing agent. In certain embodiments, the oxidant component can include from 12% to 16% of an oxidizing agent, such as hydrogen peroxide (35%) (which corresponds to 4% to about 6% H₂O₂ active).

Suitable reducing agents are those that will react with the oxidizing agent when the two components of the formulation are mixed, to generate a perceptible exothermic reaction. Suitable reducing agents should also be safe for use on human skin in the amounts used in the formulation. The reducing agent may include, for example, thiosulfate and sulfite compounds, such as sodium sulfite, sodium thiosulfate (e.g., sodium thiosulfate pentahydrate), ammonium thiosulfate, potassium thiosulfate, and thiourea. Other suitable reducing agents include compounds with a thiourea backbone, such as 1,5-diethyl-2-thiobarbituric acid or its derivatives, or ascorbic acid. Mixtures of the above reducing agents, and other suitable reducing agents, may also be used. In some embodiments, the reductant component of a shave foam composition may include from 2% to 10%, preferably from 3% to 8%, of a reducing agent.

The oxidizing agent and reducing agent are generally included in approximately stoichiometric proportions, based on the redox reaction that will occur. The predominant redox reaction of hydrogen peroxide with sodium thiosulfate is as follows:

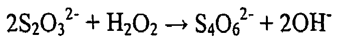

In the presence of an adequate amount of an effective catalyst, the reaction is as follows:

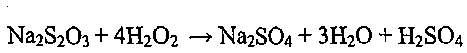

The total amount of the two agents is selected to provide a desired level of heat and duration of the exothermic reaction. Preferably, the maximum temperature obtained by the shave foam during the reaction is from about 30°C to about 60°C, and this temperature is reached from about 10 seconds to about 45 seconds after the two components are mixed (this is the temperature the shave foam reaches when the oxidant component and the reductant component of the shave foam are mixed in a beaker in stoichiometric amounts that provide a total weight of 10 grams of the shave foam; when a typical amount of from about 5 grams to about 8 grams of shave foam is applied to the skin, the actual temperature on the skin is typically from about 28°C to about 45°C). When the oxidizing agents and reducing agents described above are used, the shave foam composition generally includes from about 2% to about 10% of the oxidizing agent and from about 2% to about 10% of the reducing agent, in approximately stoichiometric proportions.

To obtain the heat profile described above, it may be advantageous to include a catalyst in the shave foam composition. The catalyst is selected to catalyze the exothermic reaction, without deleterious effects on the skin or on the properties of the shave foam. The catalyst is generally included in the reductant component of the shave foam composition. Suitable catalysts for the exothermic reaction described above include sodium molybdate (e.g., sodium molybdate dihydrate), potassium molybdate, ammonium molybdate, sodium tungstate, potassium tungstate, and mixtures thereof. The composition generally includes 0.1 % to 1.5%, preferably 0.2% to 1.0%, of the catalyst.

If the exothermic reaction generates an acid, as the reaction of the oxidizing and reducing agents discussed above will generally do, it is preferred that the composition (e.g., the reductant component) also include a neutralizing agent (a neutralizer). The neutralizing agent is selected and provided in a sufficient amount to neutralize enough of the acid so that exothermic reaction is complete and the shave foam composition will not irritate the user's skin. Preferably, substantially all of the acid is neutralized. Suitable neutralizing agents include, for example, triethanolamine, oxides (e.g., metal oxides), hydroxides (e.g., metal hydroxides), and metal carbonates, such as carbonates of alkaline metals (e.g., sodium, potassium), alkaline-earth metals (e.g., magnesium, barium), or transition metals (e.g., zinc). For example, the neutralizing agent may include calcium oxide, potassium hydroxide, sodium hydroxide, potassium bicarbonate, sodium bicarbonate or aluminum hydroxycarbonate. In some embodiments, the shave foam composition (preferably the reductant component of the shave foam composition) can include from 0.5% to 10% of such a neutralizer. For example, the reductant component can include 1% calcium oxide or 7% triethanolamine.

The shave foam composition can include additional non-ionic co-surfactants, typically in an amount of from 1% to 6%, preferably from 2% to 5%. The shave foam composition can also include additional amphoteric co-surfactants, typically in an amount of 0.1 % to 3.0%, preferably from 0.2% to 1.5%. These additional surfactants are typically included in the reductant component unless they are also determined to be stable in the presence of oxidizing agent.

Suitable non-ionic co-surfactants include the fatty esters of polyhydro alcohols (e.g. polyglyceryl oleates), polyethylene oxide fatty esters of glycerides and fatty amides, particularly the alkyl-substituted fatty amides. These surfactants will generally have from about 6 to about 100, preferably from about 20 to about 50, ethylene oxide units per molecule. Typical non-ionic co-surfactants include, for example, PEG-40 hydrogenated castor oil and decaglycerol monooleate. Suitable amphoteric surfactants include, for example, the betaines and sultaines such as cocoamidopropyl betaine, coco dimethyl carboxymethyl betaine, coco sultaine and the like. These amphoteric surfactants may tend to function as foam boosters and stabilizers, providing additional heat stability for the foam and preventing puddling. It is preferred that the composition include from 0.2% to 1.5% of an amphoteric surfactant as a foam booster. Other suitable co-surfactants that can function as foam boosters include sodium lauroyl lactylate, sodium caproyl lactylate, and short-chain alkyl polyglucosides (e.g., alkyl polyglucosides with carbon chain lengths of C12 or less, such as lauryl glucoside, capryl glucoside, or caprylyl glucoside).

Although not necessary to forming a useful shave foam composition, other cosmetic ingredients may be advantageously added to improve the application aesthetics and/or achieve other shave benefits. For example, the composition may include one or more of the following components: beard wetting agents, skin conditioning (e.g., exfoliating, moisturizing) agents (e.g., vitamin precursors and derivatives such as, for example, vitamins A, C and E, aloe, allantoin, panthenol, alpha-hydroxy acids, beta-hydroxy acids, phospholipids, triglycerides, botanical oils, amino acids), foam boosters (other than the foam-boosting co-surfactants described above), emollients (e.g., sunflower oil, fatty esters, squalane, quaternary compounds (e.g., polyquaternium-10), humectants (e.g., glycerin, sorbitol, pentylene glycol), phosphorus lipids (used, e.g., to encapsulate skin conditioning agents), fragrances, colorants, antioxidants, preservatives, and other such ingredients. In some embodiments, the reductant component of a shave foam composition can include from 0.1% to 1.5% of a fragrance.

An example of a preferred shave foam composition is one that includes cetyl alcohol as a thickener, Steareth-21 as a non-ionic surfactant, and a foaming agent with a boiling point of from about -25°C to about 10°C and/or a vapor pressure of from 310 kPa to 448 kPa (45 to 65 psig) at 21°C.

The oxidant component and the reductant component are maintained separate from each other until the product is dispensed. This may be accomplished using any dosired type of two-component packaging, e.g., as described in U.S. Patent Nos. 3,241,722; 3,454,198; and 6,250,505; and in co-pending U.S.S.N. 10/283,033, filed October 29, 2002. Generally, suitable packaging includes a pressurized container including two chambers, for example, twp bags, and at least one dispensing valve for dispensing the contents of the chambers. The two components are mixed, either automatically during actuation of the dispensing valve or manually by the user after dispensing, to form a uniform shave foam that becomes warm as the oxidizing and reducing agents react and that forms a lather upon spreading on the skin.

As will be illustrated below, the oxidant and reductant components may be formed by adding the oxidizing agent and reducing agent, respectively, to first and second shave foam bases. Preferably, the first and second shave foam bases are substantially identical. Thus, advantageously the oxidizing agent and the reducing agent, respectively, may be added to separate portions of the same shave foam base. The use of a single shave foam base to manufacture both components generally simplifies manufacturing, and may make the two components easier to mix during or after dispensing.

The shave foams described above may be formed using any suitable manufacturing process. An example of a suitable process is as follows.

Deionized water is heated to a temperature of from about 75°C to about 80°C. During the heating step, the emollient oil (e.g., mineral oil), thickeners (e.g., fatty alcohol, PEG-150 Distearate) and surfactants are added. The mixture is maintained at a temperature of from about 75°C to about 80°C with mixing for about 15 minutes. Then the mixture is allowed to cool to room temperature. During the cooling phase, at 75°C, a neutralizer is added.

To form the reductant component, active agents such as sodium thiosulfate and sodium molybdate are added to the shave foam base formed above, followed by the fragrance and dye, with mixing at from about 35°C to about 45°C.

To form the oxidant component, an aqueous solution of hydrogen peroxide is added to the shave foam base and mixed at a temperature of from about 35°C to about 40°C.

Each component is then added to a two-component aerosol can and sealed with a valve. The foaming agent, at the desired weight percentage, is then added to one or both (preferably both) components through the valve under pressure at a temperature of from about 20°C to about 30°C. It is preferable to place the oxidant component in the inner part of the can (e.g., in a bag in the can) and the reductant component in the outer part of the can. Even if there is a breach in the can, having the oxidant component in the inner part of the can will generally ensure the integrity of the can. In some embodiments, the oxidant component can be placed in a bag in the can, and the reductant component can be placed in another bag in the can that, for example, surrounds the first bag.

The following examples are intended to be illustrative and non-limiting.

### EXAMPLE 1

A shave foam composition is prepared with the following components. The shave foam composition includes a single ethoxylated alcohol as the non-ionic surfactant, and does not include any neutralizer.

| | REDUCTANT COMPONENT | OXIDANT COMPONENT |
|---|---|---|
| Ingredients | % by weight | % by weight |
| Water | 81.9 | 73.9 |
| Cetyl Alcohol | 5.0 | 5.0 |
| Steareth-21 | 2.6 | 2.6 |
| Sodium molybdate dihydrate | 0.3 | |
| Sodium thiosulfate pentahydrate | 5.7 | |
| Hydrogen peroxide (35%) | | 14.0 |
| 1,1-difluoroethane | 4.5 | 4.5 |

### EXAMPLE 2

A shave foam composition is prepared with the following components. The shave foam composition includes a single ethoxylated alcohol as the non-ionic surfactant, and does not include any neutralizer. The shave foam composition also includes an emollient.

| | REDUCTANT COMPONENT | OXIDANT COMPONENT |
|---|---|---|
| Ingredients | % by weight | % by weight |
| Water | 78.7 | 75.9 |
| Cetyl Alcohol | 6.47 | 4.2 |
| Steareth-21 | 3.43 | 2.2 |
| Sodium molybdate dihydrate | 0.30 | |
| Sodium thiosulfate pentahydrate | 6.00 | |
| Isostearyl neopentanoate | 1.50 | 2.0 |
| Hydrogen peroxide (35%) | | 11.0 |
| Isobutane/propane (A-46) | 3.6 | |
| 1,1-difluorethane | | 4.5 |

### EXAMPLE 3

A shave foam composition is prepared with the following components. The shave foam composition includes a mixture of ethoxylated alcohols as the non-ionic surfactant. The oxidant component contains a stoichiometric excess of hydrogen peroxide.

| | REDUCTANT COMPONENT | OXIDANT COMPONENT |
|---|---|---|
| Ingredients | % by weight | % by weight |
| Water | 76.96 | 70.5 |
| Steareth-2 | 2.0 | 2.0 |
| Steareth-21 | 3.5 | 3.0 |
| Cetyl Alcohol | 4.5 | 4.0 |
| PEG-150 Distearate | 1.0 | 1.0 |
| Sodium molybdate dihydrate | 0.14 | |
| Sodium thiosulfate pentahydrate | 7.0 | |
| Hydrogen Peroxide (35%) | | 16.0 |
| Calcium oxide | 1.0 | |
| Fragrance | 0.4 | |
| 1 ,1,difluoroethane and/or butane/propane (A-46) | 3.5 | 3.5 |

### EXAMPLE 4

A shave foam composition is prepared with the following components. The reductant component includes a catalyst and a CaO neutralizer, and contains a stoichiometric excess of sodium thiosulfate.

**¹ Arlacel P-135 (ICI).**

| | REDUCTANT COMPONENT | OXIDANT COMPONENT |
|---|---|---|
| Ingredients | % by weight | % by weight |
| Water | 66.9 | 73.9 |
| Mineral oil 65/75 | 9.5 | |
| PEG-30 dipolyhydroxystearate¹ | 2.3 | |
| Calcium oxide | 4.0 | |
| Cetyl Alcohol | 1.0 | 5.0 |
| Steareth-21 | | 2.6 |
| Sodium molybdate dihydrate | 0.3 | |
| Sodium thiosulfate pentahydrate | 10.0 | |
| Isostearyl neopentanoate | 1.5 | |
| Hydrogen peroxide (35%) | | 14.0 |
| Butane/propane (A-46) | 4.5 | |
| 1,1-difluoroethane | | 4.5 |

### EXAMPLE 5

The reductant component of a shave foam composition is prepared with the following components. The reductant component includes a non-ionic surfactant that is polyglyceryl fatty ester based (rather than being ethoxylated fatty alcohol based). This type of reductant component can be combined, for example, with any of the above-described oxidant components.

| | REDUCTANT COMPONENT | OXIDANT COMPONENT |
|---|---|---|
| Ingredients | % by weight | % by weight |
| Water | 79.46 | 74.4 |
| Polyaldo 10-2 palmitate | 2.5 | 2.2 |
| Cetyl alcohol | 4.5 | 3.9 |
| Cetearyl polyglucoside | 1.5 | |
| Hydrogen Peroxide (35%) | | 16.0 |
| Sodium molybdate dihydrate | 0.14 | |
| Sodium thiosulfate pentahydrate | 7.0 | |
| Calcium oxide | 1.0 | |
| Fragrance | 0.4 | |
| 1,1-difluoroethane and/or butane/propane (A-46) | 3.5 | 3.5 |

When dispensed and mixed, the formulations described above create a dense warm foam on the skin, comparable to the type of foam that is generally observed when using soap-based shave foams, but without the negative attributes of soap-based foams. Application to the skin of an amount of shave foam suitable for use in shaving (approximately 8 grams) provides a pleasant warming sensation. The foam does not collapse with the heat and lasts for the entire period of shaving.

## Claims

1. A self-heating shave foam product comprising:
a container having a first chamber and a second chamber and at least one dispensing valve for dispensing the contents of said chambers, said contents being dispersed as a foam lather;
an oxidant component in the first chamber comprising a first shave foam base and an oxidizing agent;
a reductant component in the second chamber comprising a second shave foam base and a reducing agent;
the first shave foam base and the second shave foam base each independently comprising an oil-in-water emulsion including water, a volatile foaming agent having a pressure of 276 kPa to 483 kPa (40 psig to 70 psig) at 21°C, and a water-dispersible surface active agent comprising a non-ionic surfactant;
wherein each shave foam base is substantially free of soap and ionic surfactant, and wherein the oxidant component and the reductant component each independently comprises 55% to 95% water, 1% to 6% volatile foaming agent, and 2% to 15% non-ionic surfactant

2. The shave foam product of claim 1, wherein the oxidant component and the reductant component each independently comprises 60% to 90% water, 2% to 5% volatile foaming agent, and 3% to 12% non-ionic surfactant.

3. The shave foam product of claim 1, wherein the oxidizing agent and the reducing agent are selected and present in such amount and proportion to.provide a perceptibly warm foam upon mixing of the oxidant component and the reductant component during use.

4. The shave foam product of claim 1, wherein the non-ionic surfactant comprises a fatty alcohol ethoxylate.

5. The shave foam product of claim 4, wherein the fatty alcohol ethoxylate comprises a C12-C24 hydrocarbon chain and 2 to 150 ethoxy groups.

6. The shave foam product of claim 4, wherein the non-ionic surfactant comprises a blend of fatty alcohol ethoxylates including a fatty alcohol ethoxylate having from 2 to 20 ethoxy groups and a fatty alcohol ethoxylate having from 21 to 100 ethoxy groups.

7. The shave foam product of claim 5, wherein the volatile foaming agent has a vapor pressure of from 310 kPa to 448 kPa (45 to 65 psig) at 21°C.

8. The shave foam product of claim 2, wherein the volatile foaming agent has a vapor pressure of from 310 kPa to 448 kPa (45 to 65 psig) at 21°C.

9. The shave foam product of claim 7, further comprising from about 0.2 percent to about 1.5 percent of an amphoteric surfactant.

10. The shave foam product of claim 7, wherein the first shave foam base and the second shave foam base each comprise less than about one percent of soap and ionic surfactant.

11. The shave foam product of claim 7, wherein the first shave foam base and the second shave foam base each comprise about 0 percent of soap and ionic surfactant.

12. The shave foam product of claim 7, wherein the oxidant component comprises from 2% to 10% of the oxidizing agent and the reductant component comprises from 2% to 10% of the reducing agent.

13. The shave foam product of claim 7, wherein the first shave foam base and the second shave foam base each independently comprises a fatty alcohol.

14. The shave foam product of claim 7, wherein the first shave foam base and the second shave foam base each independently comprises 2% to 8% of a fatty alcohol.

15. The shave foam product of claim 1, wherein at least one of the first shave foam base and the second shave foam base comprises at least one member selected from the group consisting of an emollient, a thickener, a lubricious water-soluble polymer, a fatty alcohol, and an amphoteric surfactant.

16. The shave foam product of claim 1, 7 or 14, wherein the first shave foam base and the second shave foam base are substantially identical.

17. The shave foam product of claim 1, wherein the oxidizing agent comprises a peroxide.

18. The shave foam product of claim 1 or 17, wherein the reducing agent is selected from the group consisting of thiosulfate and sulfite compounds, thiourea compounds, and mixtures thereof.

19. The shave foam product of claim 1, wherein at least one of the first shave foam base and the second shave foam base further comprises 1 % to 15% of an emollient.

20. The shave foam product of claim 1, wherein at least one of the first shave foam base and the second shave foam base further comprises 0.01% to 10% of a thickener.

21. The shave foam product of claim 20, wherein the thickener comprises a blend of a water-soluble thickener and a water-insoluble thickener.

22. The shave foam product of claim 1, wherein the reductant component further comprises a catalyst selected to catalyze the exothermic reaction between the oxidizing agent and the reducing agent during use.

23. The shave foam product of claim 1, wherein at least one of the first shave foam base and the second shave foam base further comprises a neutralizing agent selected to neutralize acid generated by the exothermic reaction between the oxidizing agent and the reducing agent during use.

24. The shave foam product of claim 1, wherein the oxidant component further comprises polyvinylpyrrolidone.

25. The shave foam product of claim 1, wherein at least one of the first shave foam base and the second shave foam base further comprises one or more additives selected from the group consisting of beard wetting agents, skin conditioning agents, foam boosters, emollients, humectants, fragrances, colorants, antioxidants, and preservatives.

26. The shave foam product of claim 1, wherein the shave foam product is formulated so as not to require shaking to produce a shave foam.

27. A method of using a self-heating shave foam product as defined in claim 1 comprising:
dispensing the shave foam from the container without shaking the container prior to or during dispensing;
mixing the oxidant component with the reductant component to provide an exothermic reaction; and
applying the shave foam to skin.

## Patentansprüche

1. Selbsterwärmendes Rasierschaumprodukt, umfassend:
einen Behälter mit einer ersten Kammer und einer zweiten Kammer und mindestens einem Abgabeventil zum Abgeben des Inhalts der Kammern, wobei der Inhalt als Schaum abgegeben wird;
einen Oxidationsmittelbestandteil in der ersten Kammer, umfassend eine erste Rasierschaumbasis und ein Oxidationsmittel;
einen Reduktionsmittelbestandteil in der zweiten Kammer, umfassend eine zweite Rasierschaumbasis und ein Reduktionsmittel;
wobei die erste Rasierschaumbasis und die zweite Rasierschaumbasis jeweils unabhängig eine Öl-in-Wasser-Emulsion umfassen,
einschließlich Wasser, ein flüchtiges Schaummittel mit einem Druck von 246 kPa bis 483 kPa (40 psig bis 70 psig) bei 21 °C und ein wasserdispergierbares oberflächenaktives Mittel, umfassend ein nichtionisches Tensid;
wobei jede Rasierschaumbasis im Wesentlichen frei von Seife und ionischem Tensid ist und wobei der Oxidationsmittelbestandteil und der Reduktionsmittelbestandteil jeweils unabhängig 55 % bis 95 % Wasser, 1 % bis 6 % flüchtiges Schaummittel und 2 % bis 15 % nichtionisches Tensid umfassen.

2. Rasierschaumprodukt nach Anspruch 1, wobei der Oxidationsmittelbestandteil und der Reduktionsmittelbestandteil jeweils unabhängig 60 % bis 90 % Wasser, 2 % bis 5 % flüchtiges Schaummittel und 3 % bis 12 % nichtionisches Tensid umfassen.

3. Rasierschaumprodukt nach Anspruch 1, wobei das Oxidationsmittel und das Reduktionsmittel in einer solchen Menge und einem solchen Anteil gewählt und vorhanden sind, dass sie beim Mischen des Oxidationsmittelbestandteils und des Reduktionsmittelbestandteils während des Gebrauchs einen spürbar warmen Schaum liefern.

4. Rasierschaumprodukt nach Anspruch 1, wobei das nichtionische Tensid ein Fettalkoholethoxylat umfasst.

5. Rasierschaumprodukt nach Anspruch 4, wobei das Fettalkoholethoxylat eine C12-C24-Kohlenwasserstoffkette und 2 bis 150 Ethoxygruppen umfasst.

6. Rasierschaumprodukt nach Anspruch 4, wobei das nichtionische Tensid eine Mischung von Fettalkoholethoxylaten umfasst, einschließlich ein Fettalkoholethoxylat mit 2 bis 20 Ethoxygruppen und ein Fettalkoholethoxylat mit 21 bis 100 Ethoxygruppen.

7. Rasierschaumprodukt nach Anspruch 5, wobei das flüchtige Schaummittel einen Dampfdruck von 310 kPa bis 448 kPa (45 bis 65 psig) bei 21 °C aufweist.

8. Rasierschaumprodukt nach Anspruch 2, wobei das flüchtige Schaummittel einen Dampfdruck von 310 kPa bis 448 kPa (45 bis 65 psig) bei 21 °C aufweist.

9. Rasierschaumprodukt nach Anspruch 7, ferner umfassend von etwa 0,2 Prozent bis etwa 1,5 Prozent ein amphoteres Tensid.

10. Rasierschaumprodukt nach Anspruch 7, wobei die erste Rasierschaumbasis und die zweite Rasierschaumbasis jeweils weniger als etwa ein Prozent Seife und ionisches Tensid umfassen.

11. Rasierschaumprodukt nach Anspruch 7, wobei die erste Rasierschaumbasis und die zweite Rasierschaumbasis jeweils etwa 0 Prozent Seife und ionisches Tensid umfassen.

12. Rasierschaumprodukt nach Anspruch 7, wobei der Oxidationsmittelbestandteil von 2 % bis 10 % das Oxidationsmittel umfasst und der Reduktionsmittelbestandteil von 2 % bis 10 % das Reduktionsmittel umfasst.

13. Rasierschaumprodukt nach Anspruch 7, wobei die erste Rasierschaumbasis und die zweite Rasierschaumbasis jeweils unabhängig einen Fettalkohol umfassen.

14. Rasierschaumprodukt nach Anspruch 7, wobei die erste Rasierschaumbasis und die zweite Rasierschaumbasis jeweils unabhängig zu 2 % bis 8 % einen Fettalkohol umfassen.

15. Rasierschaumprodukt nach Anspruch 1, wobei mindestens die erste Rasierschaumbasis und/oder die zweite Rasierschaumbasis mindestens ein Element, ausgewählt aus der Gruppe bestehend aus einem Weichmacher, einem Verdickungsmittel, einem schlüpfrigen wasserlöslichen Polymer, einem Fettalkohol und einem amphoteren Tensid umfasst.

16. Rasierschaumprodukt nach Anspruch 1, 7 oder 14, wobei die erste Rasierschaumbasis und die zweite Rasierschaumbasis im Wesentlichen identisch sind.

17. Rasierschaumprodukt nach Anspruch 1, wobei das Oxidationsmittel ein Peroxid umfasst.

18. Rasierschaumprodukt nach Anspruch 1 oder 17, wobei das Reduktionsmittel ausgewählt ist aus der Gruppe bestehend aus Thiosulfat- und Sulfit-Verbindungen, Thioharnstoff-Verbindungen und Mischungen davon.

19. Rasierschaumprodukt nach Anspruch 1, wobei mindestens die erste Rasierschaumbasis und/oder die zweite Rasierschaumbasis ferner zu 1 % bis 15 % einen Weichmacher umfasst.

20. Rasierschaumprodukt nach Anspruch 1, wobei mindestens die erste Rasierschaumbasis und/oder die zweite Rasierschaumbasis ferner zu 0,01 % bis 10 % ein Verdickungsmittel umfasst.

21. Rasierschaumprodukt nach Anspruch 20, wobei das Verdickungsmittel eine Mischung aus einem wasserlöslichen Verdickungsmittel und einem wasserunlöslichen Verdickungsmittel umfasst.

22. Rasierschaumprodukt nach Anspruch 1, wobei der Reduktionsmittelbestandteil ferner einen Katalysator umfasst, der ausgewählt ist, um die exotherme Reaktion zwischen dem Oxidationsmittel und dem Reduktionsmittel während des Gebrauchs zu katalysieren.

23. Rasierschaumprodukt nach Anspruch 1, wobei mindestens die erste Rasierschaumbasis und/oder die zweite Rasierschaumbasis ferner ein Neutralisationsmittel umfasst, das ausgewählt ist, um Säure zu neutralisieren, die durch die exotherme Reaktion zwischen dem Oxidationsmittel und dem Reduktionsmittel während des Gebrauchs erzeugt wird.

24. Rasierschaumprodukt nach Anspruch 1, wobei der Oxidationsmittelbestandteil ferner Polyvinylpyrrolidon umfasst.

25. Rasierschaumprodukt nach Anspruch 1, wobei mindestens die erste Rasierschaumbasis und/oder die zweite Rasierschaumbasis ferner einen oder mehrere Zusatzstoffe, ausgewählt aus der Gruppe bestehend aus Bartbenetzungsmitteln, Hautkonditioniermitteln, Schaumverstärkern, Weichmachern, Feuchthaltemitteln, Duftstoffen, Farbstoffen, Antioxidationsmitteln und Konservierungsstoffen umfasst.

26. Rasierschaumprodukt nach Anspruch 1, wobei das Rasierschaumprodukt so formuliert ist, dass es nicht geschüttelt werden muss, um einen Rasierschaum zu erzeugen.

27. Verfahren zum Gebrauch eines selbsterwärmenden Rasierschaumprodukts, wie in Anspruch 1 definiert, umfassend:
Abgeben des Rasierschaums aus dem Behälter ohne Schütteln des Behälters vor oder während der Abgabe;
Mischen des Oxidationsmittelbestandteils mit dem Reduktionsmittelbestandteil, um für eine exotherme Reaktion zu sorgen; und
Auftragen des Rasierschaums auf Haut.

## Revendications

1. Produit de type mousse à raser autochauffante comprenant :
un récipient ayant une première chambre et une deuxième chambre et au moins une soupape de distribution pour distribuer le contenu desdites chambres ; ledit contenu étant dispersé en tant que mousse ;
un composant oxydant dans la première chambre comprenant une première base de mousse à raser et un agent oxydant ;
un composant réducteur dans la deuxième chambre comprenant une deuxième base de mousse à raser et un agent réducteur ;
la première base de mousse à raser et la deuxième base de mousse à raser comprenant chacune indépendamment une émulsion huile-dans-eau incluant de l'eau, un agent moussant volatil ayant une pression de 246 kPa à 483 kPa (40 psig à 70 psig) à 21 °C, et un agent tensioactif hydrodispersible comprenant un agent tensioactif non ionique ;
dans lequel chaque base de mousse à raser est essentiellement dépourvue de savon et d'agent tensioactif ionique, et dans lequel le composant oxydant et le composant réducteur comprennent chacun indépendamment 55 % à 95 % d'eau, 1 % à 6 % d'agent moussant volatil, et 2 % à 15 % d'agent tensioactif non ionique.

2. Produit de type mousse à raser selon la revendication 1, dans lequel le composant oxydant et le composant réducteur comprennent chacun indépendamment 60 % à 90 % d'eau, 2 % à 5 % d'agent moussant volatil, et 3 % à 12 % d'agent tensioactif non ionique.

3. Produit de type mousse à raser selon la revendication 1, dans lequel l'agent oxydant et l'agent réducteur sont choisis et présents en quantité et proportion telles à fournir une mousse perceptiblement chaude lors d'un mélange du composant oxydant et du composant réducteur durant l'utilisation.

4. Produit de type mousse à raser selon la revendication 1, dans lequel l'agent tensioactif non ionique comprend un éthoxylate d'alcool gras.

5. Produit de type mousse à raser selon la revendication 4, dans lequel l'éthoxylate d'alcool gras comprend une chaîne hydrocarbonée en C12 à C24 et 2 à 150 groupes éthoxy.

6. Produit de type mousse à raser selon la revendication 4, dans lequel l'agent tensioactif non ionique comprend un mélange d'éthoxylates d'alcool gras incluant un éthoxylate d'alcool gras ayant de 2 à 20 groupes éthoxy et un éthoxylate d'alcool gras ayant de 21 à 100 groupes éthoxy.

7. Produit de type mousse à raser selon la revendication 5, dans lequel l'agent moussant volatil a une pression de vapeur allant de 310 kPa à 448 kPa (45 à 65 psig) à 21 °C.

8. Produit de type mousse à raser selon la revendication 2, dans lequel l'agent moussant volatil a une pression de vapeur allant de 310 kPa à 448 kPa (45 à 65 psig) à 21 °C.

9. Produit de type mousse à raser selon la revendication 7, comprenant, en outre, d'environ 0,2 pour cent à environ 1,5 pour cent d'un agent tensioactif amphotère.

10. Produit de type mousse à raser selon la revendication 7, dans lequel la première base de mousse à raser et la deuxième base de mousse à raser comprennent chacune moins d'environ un pour cent de savon et d'agent tensioactif ionique.

11. Produit de type mousse à raser selon la revendication 7, dans lequel la première base de mousse à raser et la deuxième base de mousse à raser comprennent chacune environ 0 pour cent de savon et d'agent tensioactif ionique.

12. Produit de type mousse à raser selon la revendication 7, dans lequel le composant oxydant comprend de 2 % à 10 % de l'agent oxydant et le composant réducteur comprend de 2 % à 10 % de l'agent réducteur.

13. Produit de type mousse à raser selon la revendication 7, dans lequel la première base de mousse à raser et la deuxième base de mousse à raser comprennent chacune indépendamment un alcool gras.

14. Produit de type mousse à raser selon la revendication 7, dans lequel la première base de mousse à raser et la deuxième base de mousse à raser comprennent chacune indépendamment 2 % à 8 % d'un alcool gras.

15. Produit de type mousse à raser selon la revendication 1, dans lequel au moins l'une parmi la première base de mousse à raser et la deuxième base de mousse à raser comprend au moins un élément choisi dans le groupe constitué d'un émollient, un épaississant, un polymère hydrosoluble glissant, un alcool gras et un agent tensioactif amphotère.

16. Produit de type mousse à raser selon la revendication 1, 7, ou 14, dans lequel la première base de mousse à raser et la deuxième base de mousse à raser sont essentiellement identiques.

17. Produit de type mousse à raser selon la revendication 1, dans lequel l'agent oxydant comprend un peroxyde.

18. Produit de type mousse à raser selon la revendication 1 ou 17, dans lequel l'agent réducteur est choisi dans le groupe constitué de composés thiosulfate et sulfite, composés thio-urée, et leurs mélanges.

19. Produit de type mousse à raser selon la revendication 1, dans lequel au moins l'une parmi la première base de mousse à raser et la deuxième base de mousse à raser comprend, en outre, 1 % à 15 % d'un émollient.

20. Produit de type mousse à raser selon la revendication 1, dans lequel au moins l'une parmi la première base de mousse à raser et la deuxième base de mousse à raser comprend, en outre, 0,01 % à 10 % d'un épaississant.

21. Produit de type mousse à raser selon la revendication 20, dans lequel l'épaississant comprend un mélange d'un épaississant hydrosoluble et d'un épaississant insoluble dans l'eau.

22. Produit de type mousse à raser selon la revendication 1, dans lequel le composant réducteur comprend, en outre, un catalyseur choisi pour catalyser la réaction exothermique entre l'agent oxydant et l'agent réducteur durant l'utilisation.

23. Produit de type mousse à raser selon la revendication 1, dans lequel au moins l'une parmi la première base de mousse à raser et la deuxième base de mousse à raser comprend, en outre, un agent neutralisant choisi pour neutraliser l'acide généré par la réaction exothermique entre l'agent oxydant et l'agent réducteur durant l'utilisation.

24. Produit de type mousse à raser selon la revendication 1, dans lequel le composant oxydant comprend, en outre, une polyvinylpyrrolidone.

25. Produit de type mousse à raser selon la revendication 1, dans lequel au moins l'une parmi la première base de mousse à raser et la deuxième base de mousse à raser comprend, en outre, un ou plusieurs additifs choisis dans le groupe constitué d'agents mouillants pour la barbe, agents de conditionnement de la peau, agents moussants, émollients, humectants, parfums, colorants, antioxydants, et conservateurs.

26. Produit de type mousse à raser selon la revendication 1, où le produit de type mousse à raser est formulé de façon à ne pas exiger d'agitation pour produire une mousse à raser.

27. Procédé d'utilisation d'un produit de type mousse à raser autochauffante selon la revendication 1 comprenant :
la distribution de la mousse à raser du récipient sans agitation du récipient avant ou durant la distribution ;
le mélange du composant oxydant avec le composant réducteur pour fournir une réaction exothermique ; et
l'application de la mousse à raser sur la peau.
